(19) **Europäisches Patentamt**
**European Patent Office**
**Office européen des brevets**

(11) **EP 4 082 999 A1**

(12) **EUROPEAN PATENT APPLICATION**
published in accordance with Art. 153(4) EPC

(43) Date of publication:
**02.11.2022 Bulletin 2022/44**

(21) Application number: **20905622.5**

(22) Date of filing: **28.12.2020**

(51) International Patent Classification (IPC):
**C07C 29/149** $^{(2006.01)}$ **C07C 35/14** $^{(2006.01)}$
**B01J 23/62** $^{(2006.01)}$

(52) Cooperative Patent Classification (CPC):
**B01J 23/62; C07C 29/149; C07C 35/14**

(86) International application number:
**PCT/KR2020/019186**

(87) International publication number:
**WO 2021/133137 (01.07.2021 Gazette 2021/26)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.12.2019 KR 20190176139**
**24.12.2020 KR 20200183549**

(71) Applicant: **Hanwha Solutions Corporation**
**Jung-gu**
**Seoul 04541 (KR)**

(72) Inventors:
• **JANG, Namjin**
**Daejeon 34128 (KR)**
• **LEE, Sun Uk**
**Daejeon 34128 (KR)**
• **KIM, Eun Jeong**
**Daejeon 34128 (KR)**
• **LEE, Jong Kwon**
**Daejeon 34128 (KR)**

(74) Representative: **Berggren Oy**
**P.O. Box 16**
**Eteläinen Rautatiekatu 10A**
**00101 Helsinki (FI)**

(54) **METHOD FOR PREPARING 1,4-CYCLOHEXANEDIMETHANOL**

(57) This invention relates to a method for preparing 1,4-cyclohexanedimethanol(CHDM), more specifically to a method for preparing 1,4-cyclohexanedimethanol having a high rate of trans isomers without an isomerization reaction step.

EP 4 082 999 A1

**Description**

## BACKGROUND OF THE INVENTION

**(a) Field of the Invention**

CROSS-REFERENCE TO RELATED APPLICATION(S)

This application claims the benefit of Korean Patent Application No. 10-2019-0176139 filed on December 27, 2019 and of Korean Patent Application No. 10-2020-0183549 filed on December 24, 2020 with the Korean Intellectual Property Office, the disclosures of which are herein incorporated by reference in their entirety.

This invention relates to method for preparing 1,4-cyclohexanedimethanol(CHDM). More specifically, this invention relates to a method for preparing 1,4-cyclohexanedimethanol having a high rate of trans isomers without an isomerization reaction step.

**(b) Description of the Related Art**

1,4-cyclohexanedimethanol(CHDM) is widely used as the raw material of medicine, synthetic resin, synthetic fiber or dye, and the like, and particularly, used as the raw material of environment-friendly polyethyleneterephthalate.

1,4-cyclohexanedimethanol exists as stereoisomers of cis and trans forms, and for higher quality product, it is required to have a higher rate of trans 1,4- cyclohexanedimethanol(trans CHDM) than cis CHDM.

In general, a method for preparing CHDM involves an isomerization reaction, by progressing an isomerization reaction of 1,4-cyclohexane dicarboxylic acid(CHDA) to increase trans content, followed by hydrogenation to CHDM, or by hydrogenating CHDA to prepare CHDM, followed by an isomerization reaction to prepare CHDM with increased trans content, and the like. However, the previous method of additionally conducting an isomerization reaction is complicated and inefficient, and requires additional production cost, and thus, is not commercially preferable.

Thus, studies on the method for preparing CHDM that can obtain high trans CHDM rate without an isomerization reaction, are under progress.

For example, International Patent Publication No. 2019-046412 attempted to increase trans CHDM content in produced CHDM, by controlling the ratio of tin and ruthenium in a hydrogenation catalyst used during the reaction of CHDA, but a fixed bed reactor containing a catalyst easily progresses crystallization during the conversion of CHDA, and at this time, catalyst performance decreases by the crystallization, and thus, desired yield and trans CHDM rate cannot be achieved.

And, Korean Registered Patent No. 10-1639487 attempted to minimize by-products by using a simplified reactor, but separately from the improvement in the purify of the product, trans CHDM rate in the product was not satisfactory.

[Patent Document]

(Patent Document 0001) International Patent Publication No. 2019-046412
(Patent Document 0002) Korean Registered Patent No. 10-1639487

SUMMARY OF THE INVENTION

In order to solve the problems of the prior art, it is an object of the invention to provide a method for preparing 1,4-cyclohexanedimethanol having a high rate of trans isomers without an isomerization reaction step, by controlling the concentration of reactant 1,4-cyclohexane dicarboxylic acid(CHDA).

According to one aspect of the invention, there is provided a method for preparing 1,4-cyclohexanedimethanol comprising steps of:

supplying a reaction solution comprising 1,4-cyclohexane dicarboxylic acid(CHDA) comprising cis isomers and trans isomers, a hydrogenation catalyst, and water to a reactor equipped with a stirrer;
supplying hydrogen gas to the reactor in which the reaction solution has been supplied; and
conducting a hydrogenation reaction by stirring the stirrer of the reactor, to prepare 1,4-cyclohexanedimethanol(CHDM),
wherein the 1,4-cyclohexane dicarboxylic acid(CHDA) comprising cis isomers and trans isomers is included in the amount of 5 to 30 wt%, based on the total weight of the 1,4-cyclohexane dicarboxylic acid and water.

According to another aspect of the invention, there is provided a composition comprising 1,4-cyclohexanedimethanol prepared by the method.

According to the preparation method of 1,4-cyclohexanedimethanol of the invention, 1,4-cyclohexanedimethanol having a high trans isomer rate can be prepared by controlling the concentration of 1,4-cyclohexane dicarboxylic acid, even if a hydrogenation reaction is progressed using 1,4-cyclohexane dicarboxylic acid as starting material, without conducting an isomerization reaction.

According to the preparation method, additional isomerization step is not conducted, and thus, the process may be simplified and economical, and trans isomer rate of prepared CHDM is high, and thus, when used as the raw material of polymer, property improvement can be expected.

DETAILED DESCRIPTION OF THE EMBODIMENTS

The terms used herein are only to explain specific embodiments, and are not intended to limit the invention. A singular expression includes a plural expression thereof, unless it is expressly stated or obvious from the context that such is not intended. As used herein, the terms "comprise" , "equipped" or "have" , etc. are intended to designate the existence of practiced characteristic, number, step, constructional element or combinations thereof, and they are not intended to preclude the possibility of existence or addition of one or more other characteristics, numbers, steps, constructional elements or combinations thereof.

Although various modifications can be made to the invention and the invention may have various forms, specific examples will be illustrated and explained in detail below. However, it should be understood that these are not intended to limit the invention to specific disclosure, and that the invention includes all the modifications, equivalents or replacements thereof without departing from the spirit and technical scope of the invention.

Hereinafter, a method for preparing 1,4-cyclohexanedimethanol according to specific embodiments of the invention will be explained in detail.

The method for preparing 1,4-cyclohexanedimethanol of the invention comprises steps of: supplying a reaction solution comprising 1,4-cyclohexane dicarboxylic acid(CHDA) comprising cis isomers and trans isomers, a hydrogenation catalyst, and water to a reactor equipped with a stirrer; supplying hydrogen gas to the reactor in which the reaction solution has been supplied; and conducting a hydrogenation reaction by stirring the stirrer of the reactor, to prepare 1,4-cyclohexanedimethanol(CHDM), wherein the 1,4-cyclohexane dicarboxylic acid(CHDA) comprising cis isomers and trans isomers is included in the amount of 5 to 30 wt%, based on the total weight of the 1,4-cyclohexane dicarboxylic acid and water.

In case 1,4-cyclohexane dicarboxylic acid is hydrogenated in the presence of a hydrogenation catalyst to prepare 1,4-cyclohexanedimethanol, 1,4-cyclohexanedimethanol obtained as the reaction product is in the form of a mixture of cis CHDM and trans CHDM.

Previously, in order to prepare 1,4-cyclohexanedimethanol having a high content of trans isomers, an isomerization reaction step for converting cis isomers into trans isomers was necessarily involved. For example, using 1,4-cyclohexane dicarboxylic acid as the raw material of an isomerization reaction, 1,4-cyclohexane dicarboxylic acid having increased trans content was obtained, and then, it was used again as the raw material of a hydrogenation reaction to prepare 1,4-cyclohexanedimethanol having high trans content, or raw material 1,4-cyclohexane dicarboxylic acid was first hydrogenated, and then, obtained 1,4-cyclohexanedimethanol was subjected to an isomerization reaction to prepare 1,4-cyclohexanedimethanol having increased trans content. However, in case 1,4-cyclohexanedimethanol is prepared by the previous method, due to the additional isomerization process, the process is complicated and inefficient, and additional production cost is required, which is not commercially preferable.

However, the method for preparing 1,4-cyclohexanedimethanol according to one embodiment of the invention can prepare 1,4-cyclohexanedimethanol having high trans isomer content without conducting an isomerization reaction for converting cis isomers into trans isomers, by controlling the concentration of reactant 1,4-cyclohexane dicarboxylic acid.

When progressing a hydrogenation reaction of a compound having cis/trans isomers, it is generally expected that cis and trans ratios of the reactant and product before and after the reaction may be maintained without significant change. Even if the concentration of the reactant and reaction temperature are increased, only improvement in the reaction speed may be anticipated, and commonly, change in isomer ratio of the hydrogenation reaction product is not expected.

However, according to one embodiment of the invention, when the concentration of 1,4-cyclohexane dicarboxylic acid is within a specific range, trans isomer content of the product 1,4-cyclohexanedimethanol unexpectedly increases without additional isomerization reaction step. It is believed that the isomerization speed varies according to the concentration of 1,4-cyclohexane dicarboxylic acid, and at specific concentrations, the isomerization speed increases and a time for reaching the equilibrium of trans/cis isomer ratio is shortened.

More specifically, in the method for preparing 1,4-cyclohexanedimethanol according to one embodiment of the invention, a reaction solution comprising 1,4-cyclohexane dicarboxylic acid(CHDA) comprising cis isomers and trans isomers, a hydrogenation catalyst, and water is supplied to a reactor equipped with a stirrer

The 1,4-cyclohexane dicarboxylic acid is included in the amount of 5 to 30 wt%, based on the total weight of the 1,4-cyclohexane dicarboxylic acid and water. More specifically, the content of the 1,4-cyclohexane dicarboxylic acid may be 5 wt% or more, 7 wt% or more, or 10 wt% or more, and 30 wt% or less, or 25 wt% or less, or 23 wt% or less, based on the total amount of ,4-cyclohexane dicarboxylic acid and water.

If the amount of 1,4-cyclohexane dicarboxylic acid is less than 5 wt%, based on the total amount of 1,4-cyclohexane dicarboxylic acid and water, contact between reactant and catalyst may decrease, and thus, a reaction speed may decrease, or the rate of trans isomers may decrease in produced CHDM, and if it is greater than 30 wt%, solubility of 1,4-cyclohexane dicarboxylic acid may be lowered, and thus, productivity may decrease, and crystal of the reactant and catalyst amount may increase, thus causing a difficulty in feeding of slurry.

Wherein, although the rates of cis isomers and trans isomers of the reactant 1,4-cyclohexane dicarboxylic acid is not limited, the 1,4-cyclohexane dicarboxylic acid may have trans isomer rate of 60 wt% or more, or 62 wt% or more, or 65 wt% or more, or 67 wt% or more, or 70 wt% or more, and the upper limit of the trans isomer rate is not limited, but for example, it may be 80 wt% or less, or 78 wt% or less, or 75 wt% or less.

According to one embodiment of the invention, the hydrogenation catalyst may comprise one or more metals selected from the group consisting of palladium(Pd), rhodium(Rh), and ruthenium(Ru), and one or more metals selected from the group consisting of tin(Sn), iron(Fe), rhenium(Re), and gallium(Ga), as active components.

Preferably, the hydrogenation catalyst may comprise ruthenium(Ru) and tin(Sn) as active components. More preferably, the active components of the hydrogenation catalyst may consist only of ruthenium(Ru) and tin(Sn), and other active components may not be included.

According to one embodiment of the invention, the amount of the active components of the hydrogenation catalyst may be appropriately controlled according to the content of reactant CHDA. Specifically, as the content of the hydrogenation catalyst based on CHDA is higher, a reaction speed increases, and thus, the hydrogenation catalyst may be added in such an amount that the weight ratio of the hydrogenation catalyst and CHDA may become 0.01:1 or more.

However, if the content of the hydrogenation catalyst based on CHDA is above a certain level, the reaction speed increase effect may be insignificant compared to the amount used, thus decreasing reaction efficiency, and thus, the hydrogenation catalyst may be more specifically added in such an amount that the weight ratio of the hydrogenation catalyst and CHDA may fulfill 0.01:1 to 3:1.

Considering the reaction speed improvement effect according to control of the weight ratio of the hydrogenation catalyst and CHDA, it may be more preferable to add the hydrogenation catalyst in such an amount that the weight ratio of the hydrogenation catalyst and CHDA may become 0.01:1 to 3:1, or 0.1:1 to 3:1, or 0.1:1 to 2:1.

However, the scope of the invention is not limited by the above weight ratio, and the rate of a catalyst may be appropriately controlled according to detailed reaction conditions, and the kind of a reactor.

Such a hydrogenation catalyst may be supported on a carrier, and as the carrier, those known in the art may be used without limitations. Specifically, carbon, zirconia($ZrO_2$), titania($TiO_2$), alumina($Al_2O_3$), or so;oca($SiO_2$), and the like may be used.

According to one embodiment of the invention, in case the hydrogenation catalyst comprises ruthenium(Ru) and tin(Sn) as active components, ruthenium(Ru) and tin(Sn) may be included respectively in an amount of 1 to 20 parts by weight, or 1 to 10 parts by weight, or 3 to 8 parts by weight, based on 100 parts by weight of the carrier.

When carbon is used as the carrier, although not limited, at least one selected from the group consisting of activated carbon, carbon black, graphite, graphene, OMC (ordered mesoporous carbon) and carbon nanotube may be used.

Preferably, it may be carbon black having a high rate of mesopores in the total pores, and specifically, the activated carbon may be SXULTRA, CGSP, PK1-3, SX 1G, DRACO S51HF, CA-1, A-51, GAS 1240 PLUS, KBG, CASP and SX PLUS, and the like, and the carbon black may be BLACK PEARLS®, ELFTEX®, VULCAN®, MOGUL®, MONARCH®, EMPEROR®, and REGAL®, and the like, but not limited thereto.

Wherein, according to the invention, in the carbon carrier, the volume fraction of mesopores having sizes of 2 to 50 nm in the total pores may be 50% or more. Preferably, in the carbon carrier, the volume fraction of mesopores in the total pores may be 70% or more, and more preferably, in the carbon carrier, the volume fraction of mesopores in the total pores may be 75% or more.

Wherein, if the volume fraction of mesopores is less than 50%, there may be a problem in terms of a speed of microscopic transfer of reactant and product in the carbon carrier, and if the average size of the pores is greater than 50 nm, the physical strength of the carrier may be weak, and thus, the above ranges are preferable.

And, according to the invention, the carbon comprises ordered mesoporous carbon(OMC) having specific surface area(BET) of 100 to 1,500 m2/g. Preferably, the carbon may comprise ordered mesoporous carbon(OMC) having specific surface area(BET) of 200 to 1,000 $m^2$/g. Wherein, if the specific surface area of carbon is less than 100 m2/g, it may be difficult to highly disperse active metals(Ru, Sn), and if the specific surface area of carbon is greater than 1,500

m$^2$/g, the fraction of mesopores may decrease, and thus, the above ranges are preferable.

And, in some cases, the carbon carrier of the catalyst according to the invention comprises an appropriate fraction of micropores, besides mesopores, and preferably, the volume fraction of micropores may be 0 to 25% in the total pores. Wherein, in case the volume fraction of micropores is greater than 25%, there may be a problem in terms of a speed of microscopic transfer of reactant and product in the carbon carrier, and thus, the above range is preferable.

In case the hydrogenation catalyst is supported on a carrier, the amount of the active components of the hydrogenation catalyst may be preferably 20 parts by weight or less, or 15 parts by weight or less, or 10 parts by weight or less, and 1 part by weight or more, or 3 parts by weight or more, based on 100 parts by weight of the carrier. If the amount of the hydrogenation catalyst is too large based on 100 parts by weight of the carrier, a reaction may rapidly progress on the catalyst surface, and during this process, side reactions may also increase, thus rapidly increasing by-products, and if it is too small, due to insufficient catalyst amount, the yield of the hydrogenation reaction may be lowered, and thus, the above range is preferable.

The method for preparing 1,4-cyclohexanedimethanol according to one embodiment of the invention may be conducted using a reactor comprising a stirrer, a raw material inlet, a metal sintered filter, and a product outlet.

For example, the stirrer may be a gas-induced type stirrer comprising a gas inlet, a gas passage, an impeller and jet orifices.

More specifically, the stirrer is provided in the up and down direction of the reactor, and the upper part may be provided with a gas inlet for inhaling gas, namely, hydrogen gas by centrifugal force. The hydrogen gas inhaled in the gas inlet is passed to the lower part of the reactor through the gas passage. The hydrogen gas passed to the lower part of the reactor is sprayed and fed into the reaction solution through plural jet orifices of the stirrer, thus conducting a hydrogenation reaction. The jet orifice may be positioned at the lower part, on the side, or both at the lower part and on the side of the stirrer.

As such, as a hydrogenation reaction is conducted while spraying and mixing hydrogen gas inhaled through the gas inlet into the reaction solution through the plural jet orifices of the stirrer, hydrogenation reaction speed may increase.

And, since the stirrer comprises an impeller stirring the reaction solution, gas holdup and surface area per unit volume may increase. Thus, a hydrogenation reaction speed in the reactor may increase.

The impeller may be arranged in multi stages at the rotation axis of the stirrer.

Alternatively, according to another embodiment, only an impeller having jet orifices may be provided at the lower part of the stirrer, and additional impellers may not be provided.

The rotation axis may be operated by a driving motor equipped outside.

The lower part of the reactor may be connected to the raw material inlet, and raw materials, namely, 1,4-cyclohexane dicarboxylic acid, solvents, and hydrogen gas may be introduced therein.

Meanwhile, the reactor may comprise a metal sintered filter for filtering a catalyst from the product, and a product outlet, wherein the metal sintered filter may be connected to the product outlet and installed. And, the metal sintered filter may be connected to the product outlet and provided outside of the reactor. The metal sintered filter may effectively filter catalyst components remaining in the product.

Next, in the reactor where the reaction solution has been introduced, hydrogen gas is fed.

The hydrogenation reaction may be conducted in liquid phase or gas phase. According to one embodiment of the invention, a hydrogenation reaction may be progressed while 1,4-cyclohexane dicarboxylic acid is a liquid phase dissolved in a solvent such as water, and hydrogen is a gas phase,

Next, by stirring the stirrer of the reactor to conduct a hydrogenation reaction, 1,4-cyclohexanedimethanol is prepared.

Although the hydrogenation reaction conditions are not specifically limited herein, for example, a reaction temperature may be 230°C or more, and 300°C or less, or 280 °C or less, or 270°C or less. If the reaction temperature is less than 230 °C, contact with a catalyst may decrease or the temperature may not fall within a temperature at which the catalyst is activated, and thus, a reaction speed may decrease, or the content of trans isomers in produced CHDA may decrease, and if the reaction temperature is greater than 300 °C, by-products may rapidly increase, and catalyst life may be also influenced, and thus, the above range is preferable.

And, a reaction pressure may be 50 bar or more, or 80 bar or more, and 220 bar or less, or 200 bar or less, or 180 bar or less. If the reaction pressure is less than 50 bar, a reaction may not sufficiently occur, and thus, an excessive amount of catalyst may be consumed, and residence time may be too lengthened, thus causing a lot of problems such as by-product increase, and if the reaction pressure is greater than 220 bar, excessive energy may be required during process operation, and the production cost of equipment such as a reactor may significantly increase, and thus, the above range is preferable.

Since the reaction pressure is a pressure established by hydrogen gas supplied, it may be controlled according to the amount of hydrogen gas supplied.

During the hydrogenation reaction, a stirring process is conducted, and the reaction efficiency of the hydrogenation reaction may be increased through control of the stirring speed. Specifically, the stirring process may be conducted such that the surface area per unit volume of hydrogen gas bubbles may become 15 $m^2/m^3$ or more, more specifically, 50 $m^2/m^3$ or more, or 100 $m^2/m^3$ or more, or 150 $m^2/m^3$ or more, or 200 $m^2/m^3$ or more, or 300 $m^2/m^3$ or more.

As long as the surface area per unit volume meets a certain level, for example, 15 $m^2/m^3$ or more, a reaction speed is slower than a speed at which hydrogen gas is dissolved, and thus, a reaction speed may not be significantly influenced. Thus, the upper limit of the surface area per unit volume is not specifically limited as long as it meets 15 $m^2/m^3$ or more, but considering the energy efficiency of a reactor, it is preferably 500 $m^2/m^3$ or less.

Meanwhile, the stirring process may be conducted using the stirrer of the reactor as explained above.

It may be more preferable in terms of process efficiency that the reaction is conducted for 1 to 10 hours under conditions fulfilling all the hydrogenation reaction conditions as described above.

In the reaction product obtained after the reaction, CHDM comprising cis isomers and trans isomers, solvent water, and a catalyst, and the like are included, and it may be used as the reactant of various reactions. It may be used, after removing by-products, solvents and catalysts, and the like included in the reaction product by a purification process, as necessary.

According to one embodiment of the invention, in the reaction product of above step, the amount of CHDM comprising cis isomers and trans isomers may be 5 to 30 wt%, based on the total amount of the reaction product of the step. More specifically, it may be 5 wt% or more, or 7 wt% or more, or 10 wt% or more, and 30 wt% or less, or 25 wt% or less, or 23 wt% or less.

According to one embodiment of the invention, in case a mixed solution comprising CHDA, a hydrogenation catalyst and water, wherein the amount of CHDA is 5 to 30 wt%, 7 to 30 wt%, 7 to 25 wt%, 7 to 23 wt%, or 10 to 23 wt%, based on total weight of 1,4-cyclohexane dicarboxylic acid and water, is subjected to a hydrogenation reaction to prepare CHDM, the rate of trans isomers in the prepared CHDM may be 63 wt% or more, or 65 wt% or more, or 67 wt% or more, or 69 wt% or more, or 70 wt% or more, and the upper limit of trans isomer rate is not limited, but for example, it may be 99 wt% or less, or 95 wt% or less, or 90 wt% or less, or 85 wt% or less.

Thus, 1,4-cyclohexanedimethanol finally obtained by the preparation method of the invention has excellent trans isomer content of 63 wt% or more, and thus, can be usefully used as the raw material for preparing high quality products, without additional isomerization process.

According to another embodiment of the invention, there is provided a composition comprising 1,4-cyclohexanedimethanol prepared by the method for preparing 1,4-cyclohexanedimethanol.

In order to immediately use 1,4-cyclohexanedimethanol as the raw material or reactant of other processes without an isomerization process, the content of trans isomers in 1,4-cyclohexanedimethanol should be 63 wt% or more.

The composition comprising 1,4-cyclohexanedimethanol of the invention may have very high trans isomer content such as 63 wt% or more, 65 wt% or more, 67 wt% or more, 69 wt% or more, or 70 wt% or more in 1,4-cyclohexanedimethanol. And, although the upper limit of the trans isomer rate is not limited, but for example, it may be 99 wt% or less, 95 wt% or less, 90 wt% or less, or 85 wt% or less.

The composition of one embodiment may be used as the raw material of medicine, synthetic resin, synthetic fiber or dye.

Hereinafter, the invention will be explained in more detail through examples for better understanding of the invention. However, these examples are presented only as the illustrations of the invention, and the invention is not limited thereby.

**<Example>**

**Example 1**

A reactor equipped with a gas-induced type stirrer was prepared.

In the reactor, 550g of CHDA (trans CHDA rate in CHDA: 68 wt%), 2,100 g of distilled water solvent, and 152 g of a catalyst(ruthenium-tin/carbon catalyst, comprising 5 parts by weight of ruthenium, and 5.8 parts by weight of tin, based on 100 parts by weight of a carbon carrier) were introduced as reactants, purged twice with nitrogen of 5 bar, and purged twice with hydrogen of 5 bar, and then, the temperature was raised to 230 °C while stirring at 50 rpm under hydrogen atmosphere (about 14-15 bar).

After reaching the reaction temperature, hydrogen was introduced to the reaction pressure of 100 bar, and then, the stirring speed was increased, and a reaction was conducted for 6 hours while maintaining the surface area per unit volume of hydrogen gas bubbles at 300 to 450 $m^2/m^3$.

**Example 2**

The same procedure as the step 1 of Example 1 was conducted, except that 550g of CHDA having trans CHDA rate of 21 wt% in CHDA was used in Example 1.

### Example 3

The same procedure as the step 1 of Example 1 was conducted, except that 158 g of CHDA having trans CHDA rate of 21 wt% in CHDA was used in Example 1.

### Comparative Example 1

The same procedure as the step 1 of Example 1 was conducted, except that 34 g of CHDA having trans CHDA rate of 21 wt% in CHDA was used in Example 1.

### <Experimental Example>

For the Examples, changes in CHDM yield, conversion, selectivity were calculated, and shown in the following Table 1.

$$Conversion = mole\ number\ of\ reacted\ CHDA\ /\ mole\ number\ of\ supplied\ CHDA$$

$$Selectivity = mole\ number\ of\ produced\ CHDM\ /\ mole\ number\ of\ reacted\ CHDA$$

$$Yield = conversion \times selectivity$$

And, trans CHDM content in the product CHDM was analyzed with gas chromatography(GC, column: HP-5, detector: FID).

[Table 1]

|  | CHDA content* (wt%) | trans CHDM content in the product CHDM (wt%) | CHDA conversion rate (%) | CHDM selectivit y (%) | CHDM yield (%) |
|---|---|---|---|---|---|
| Example 1 | 21 | 75 | 99 | 96 | 95 |
| Example 2 | 21 | 68 | 99 | 96 | 95 |
| Example 3 | 7 | 67 | 99.9 | 96.5 | 95.5 |
| Comparati ve Example 1 | 1.6 | 56 | 99 | 93 | 92 |
| * The content of CHDA means the content(wt%) based on the total amount of CHDA and water. | | | | | |

Referring to Table 1, it was confirmed that in the case of Examples 1 to 3 comprising a certain concentration of CHDA, trans CHDM content in the hydrogenation reaction product CHDM was greater than 68 wt%, and thus, very high content of trans isomers were produced.
However, in the case of Comparative Example 1 comprising 1.6 wt% of CHDA, trans CHDM was included only in the content of 56 wt% in the product CHDM, and thus, sufficient trans isomers could not be produced.

## Claims

**1.** A method for preparing 1,4-cyclohexanedimethanol comprising steps of:

supplying a reaction solution comprising 1,4-cyclohexane dicarboxylic acid(CHDA) comprising cis isomers and trans isomers, a hydrogenation catalyst, and water to a reactor equipped with a stirrer;
supplying hydrogen gas to the reactor in which the reaction solution has been supplied; and
conducting a hydrogenation reaction by stirring the stirrer of the reactor, to prepare 1,4-cyclohexanedimethanol(CHDM),
wherein the 1,4-cyclohexane dicarboxylic acid(CHDA) comprising cis isomers and trans isomers is included in the amount of 5 to 30 wt%, based on the total weight of the 1,4-cyclohexane dicarboxylic acid and water.

2. The method for preparing 1,4-cyclohexanedimethanol according to claim 1, wherein the 1,4-cyclohexane dicarboxylic acid(CHDA) is included in an amount of 7 to 23 wt%, based on the total weight of the 1,4-cyclohexane dicarboxylic acid and water.

3. The method for preparing 1,4-cyclohexanedimethanol according to claim 1, wherein the step of conducting a hydrogenation reaction is conducted at a temperature of 230 to 300 °C.

4. The method for preparing 1,4-cyclohexanedimethanol according to claim 1, wherein the 1,4-cyclohexane dicarboxylic acid comprises 60 wt% or more of trans isomers.

5. The method for preparing 1,4-cyclohexanedimethanol according to claim 1, wherein the hydrogen gas is supplied at a pressure of 50 to 220 bar.

6. The method for preparing 1,4-cyclohexanedimethanol according to claim 1, wherein the hydrogenation catalyst comprises one or more metals selected from the group consisting of palladium(Pd), rhodium(Rh), and ruthenium(Ru), and one or more metals selected from the group consisting of tin(Sn), iron(Fe), rhenium(Re), and gallium(Ga).

7. The method for preparing 1,4-cyclohexanedimethanol according to claim 6, wherein the hydrogenation catalyst comprises ruthenium(Ru) and tin(Sn).

8. The method for preparing 1,4-cyclohexanedimethanol according to claim 1, wherein the stirring is conducted such that a surface area per unit volume of hydrogen gas bubbles becomes 15 $m^2/m^3$ or more.

9. The method for preparing 1,4-cyclohexanedimethanol according to claim 1, wherein the 1,4-cyclohexanedimethanol comprises 63 wt% or more of trans isomers.

10. A composition comprising 1,4-cyclohexanedimethanol prepared by the method of claim 1.

<div align="center">**INTERNATIONAL SEARCH REPORT**</div>

| International application No. |
| --- |
| **PCT/KR2020/019186** |

**A. CLASSIFICATION OF SUBJECT MATTER**

**C07C 29/149**(2006.01)i; **C07C 35/14**(2006.01)i; **B01J 23/62**(2006.01)i

According to International Patent Classification (IPC) or to both national classification and IPC

**B. FIELDS SEARCHED**

Minimum documentation searched (classification system followed by classification symbols)

C07C 29/149(2006.01); B01J 23/44(2006.01); C07C 31/27(2006.01); C07C 35/14(2006.01); C07C 51/353(2006.01); C07C 51/36(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 1,4-사이클로헥산디메탄올(1,4-cyclohexane dimethanol), 1,4-사이클로헥산디카르복실산(1,4-cyclohexane dicarboxylic acid), 물(water, H2O), 주석(tin, Sn), 루테늄 (Ruthenium, Ru)

**C. DOCUMENTS CONSIDERED TO BE RELEVANT**

| Category* | Citation of document, with indication, where appropriate, of the relevant passages | Relevant to claim No. |
| --- | --- | --- |
| X | US 6495730 B1 (KONISHI, M. et al.) 17 December 2002 (2002-12-17) See column 13, lines 54-64; column 17, lines 49 and 50; column 22, example 2; and column 30, example 13. | 1-10 |
| A | WO 2015-102892 A1 (EASTMAN CHEMICAL COMPANY) 09 July 2015 (2015-07-09) See paragraphs [0034] and [0047]. | 1-10 |
| A | KR 10-2004-0047974 A (MITSUBISHI CHEMICAL CORPORATION) 05 June 2004 (2004-06-05) See claims 1-17. | 1-10 |
| A | JP 2014-177422 A (MITSUBISHI CHEMICALS CORP.) 25 September 2014 (2014-09-25) See paragraphs [0033]-[0058]. | 1-10 |
| A | JP 2002-069016 A (MITSUBISHI CHEMICALS CORP.) 08 March 2002 (2002-03-08) See claim 1; and paragraph [0014]. | 1-10 |

☑ Further documents are listed in the continuation of Box C.　　☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **07 April 2021** | **07 April 2021** |

| Name and mailing address of the ISA/KR | Authorized officer |
| --- | --- |
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| International application No. |
|---|
| **PCT/KR2020/019186** |

### C.    DOCUMENTS CONSIDERED TO BE RELEVANT

| Category* | Citation of document, with indication, where appropriate, of the relevant passages | Relevant to claim No. |
|---|---|---|
| A | KR 10-2018-0035585 A (LOTTE CHEMICAL CORPORATION) 06 April 2018 (2018-04-06)<br>See claims 1-20. | 1-10 |

Form PCT/ISA/210 (second sheet) (July 2019)

**INTERNATIONAL SEARCH REPORT**
**Information on patent family members**

International application No.

**PCT/KR2020/019186**

| Patent document cited in search report | | | Publication date (day/month/year) | Patent family member(s) | | | Publication date (day/month/year) |
|---|---|---|---|---|---|---|---|
| US | 6495730 | B1 | 17 December 2002 | AU | 7318900 | A | 24 April 2001 |
| | | | | CN | 1141180 | C | 10 March 2004 |
| | | | | CN | 1374884 | A | 16 October 2002 |
| | | | | EP | 1232789 | A1 | 21 August 2002 |
| | | | | JP | 2001-157840 | A | 12 June 2001 |
| | | | | JP | 4472108 | B2 | 02 June 2010 |
| | | | | KR | 10-0460371 | B1 | 08 December 2004 |
| | | | | KR | 10-2002-0040809 | A | 30 May 2002 |
| | | | | TW | 537926 | B | 21 June 2003 |
| | | | | WO | 01-21306 | A1 | 29 March 2001 |
| WO | 2015-102892 | A1 | 09 July 2015 | CN | 105849073 | A | 10 August 2016 |
| | | | | CN | 105849073 | B | 17 July 2018 |
| | | | | US | 2015-0183699 | A1 | 02 July 2015 |
| | | | | US | 2016-0229774 | A1 | 11 August 2016 |
| | | | | US | 9346737 | B2 | 24 May 2016 |
| KR | 10-2004-0047974 | A | 05 June 2004 | CN | 1310868 | C | 18 April 2007 |
| | | | | CN | 1608042 | A | 20 April 2005 |
| | | | | CN | 1935773 | A | 28 March 2007 |
| | | | | EP | 1449822 | A1 | 25 August 2004 |
| | | | | EP | 1449822 | B1 | 23 February 2011 |
| | | | | JP | 2003-128622 | A | 08 May 2003 |
| | | | | JP | 2004-043426 | A | 12 February 2004 |
| | | | | JP | 2010-163439 | A | 29 July 2010 |
| | | | | JP | 3858666 | B2 | 20 December 2006 |
| | | | | JP | 4513256 | B2 | 28 July 2010 |
| | | | | KR | 10-0943872 | B1 | 24 February 2010 |
| | | | | US | 2005-0014973 | A1 | 20 January 2005 |
| | | | | US | 2008-0051600 | A1 | 28 February 2008 |
| | | | | US | 7420086 | B2 | 02 September 2008 |
| | | | | US | 7595423 | B2 | 29 September 2009 |
| | | | | WO | 03-035597 | A1 | 01 May 2003 |
| JP | 2014-177422 | A | 25 September 2014 | JP | 6051980 | B2 | 27 December 2016 |
| JP | 2002-069016 | A | 08 March 2002 | None | | | |
| KR | 10-2018-0035585 | A | 06 April 2018 | KR | 10-1917366 | B1 | 09 November 2018 |

Form PCT/ISA/210 (patent family annex) (July 2019)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020190176139
- KR 1020200183549
- WO 2019046412 A
- KR 101639487